(19) Europäisches Patentamt
European Patent Office
Office européen des brevets

(11) Publication number: 0 113 998 A2

# (12) EUROPEAN PATENT APPLICATION

(21) Application number: **83307847.0**

(22) Date of filing: **22.12.83**

(51) Int. Cl.³: **A 61 K 31/575** // (A61K31/575, 31/57)

(30) Priority: **22.12.82 US 452303**

(43) Date of publication of application: **25.07.84 Bulletin 84/30**

(84) Designated Contracting States: **AT BE DE FR GB IT LU NL**

(71) Applicant: **HERPES PHARMACEUTICAL, INC., 1575 I Street, North West 7th Floor, Washington, D.C. 20006 (US)**

(72) Inventor: **Fareed, George, 1200 South Manning Avenue No. 13, Los Angeles California 90024 (US)**

(74) Representative: **Sheard, Andrew Gregory et al, Kilburn & Strode 30, John Street, London WC1N 2DD (GB)**

(54) **Composition and treatment for herpes simplex viral infections.**

(57) A composition for the treatment of infection comprises an antivirally effective amount of a compound of the formula (I):

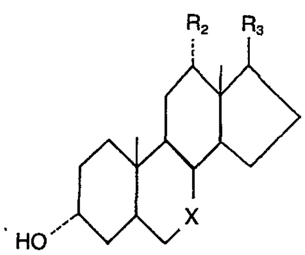

(I)

wherein X is $CH-R_1$ or $C1=O$;

wherein $R_1$ and $R_2$ are the same or different and each is H or OH;

$R_3$ is $CH_3-\overset{|}{C}H\sim CH_2)_n-COY$;
n is $OR_4$ or $NR_5R_6$, where
$R_4$ is H, a $C_1-C_{12}$ branched or unbranched alkyl group or a pharmaceutically acceptable cation;
$R_5$ and $R_6$ are the same or different and each is H, unsubstituted $C_1-C_{12}$ branched or unbranched alkyl, or branched or unbranched $C_1-C_{12}$ alkyl substituted by $-CO_2R_4$ or by $-SO_3R_4$;
or $R_5$ and $R_6$ together with the nitrogen atom form a heterocyclic ring.

Such a compound may be present in conjunction with other activated compounds.

EP 0 113 998 A2

# Composition and Treatment for Herpes Simplex Viral Infections

The present invention relates to a treatment for viral infections caused by Herpes Simplex Virus types 1 or 2.

Herpes simplex virus types 1 and 2 (hereinafter "HSV-1" and HSV-2) causes a diverse number of infections in humans. Acute disseminated primary infection, chronic infection with continued or intermittent virus shedding (herpes keratitis and herpes labialis) and clinical reactivation occur.

Herpes simplex virus encephalitis is one of the most frequent endemic encephalitis in the United States. HSV-2 is the second-most frequently transmitted venereal disease (after gonorrhea). It has also been associated with carcinoma of the cervix (See, for example, Martin Lerner, Chapter 193, "Infections with Herpes Simplex Virus" in Harrison's Principles of Internal Medicine, 9th Ed., McGraw-Hill, 1980, pp. 847-851, from which these and the following comments are taken, and which is herein incorporated by reference).

Primary infection with HSV-1 causes acute gingivostomatitis, rhinitis, keratoconjunctivitis, meningoencephalitis, eczema herpeticum and traumatic herpes, including herpetic whitlow and generalized cutaneous herpes simplex in burned patients. The incubation period is 2 to 12 days, averaging 6 or 7

days. The route of infection is contact with infected skin or mucosal surfaces.

Occasionally, and for ill-defined reasons, HSV-1 begins an ascent from the respiratory epithelium of the nose up the olfactory tract to reach the frontal and temporal areas of the brain. An often fatal or severely damaging necrotizing encephalitis results. Patients of any age, either sex, or any socioeconomic status may be affected.

Infection with HSV-2 is usually sexually transmitted. This infection is the most common cause of genital vesicles and/or ulcers found in women, and is second only to primary syphilis as the cause of such lesions in males. Teenagers make up 1/4 to 1/2 of patients with genital herpetic infections.

Idoxuridine (5-iodo-2' -deoxyuridine, IDU), trifluridine and adenine arabinoside (9-beta-D- arabinofuranosyladenine, Ara-A) are effective topically in herpes simplex virus keratitis, as is acyclovir (9-(2-hydroxyethoxymethyl)guanine). For herpetic keratitis topical IDU is applied in a 0.1% solution every hour during the day and every 2 hours during the night. A simpler means of application is a 0.5% ointment 4-5 times a day.

There have been, prior to this invention, very few controlled clinical trials to support the efficacy of any local preparation in the treatment of herpes labialis or genital herpes simplex virus infection.

Intravenous Ara-A has also been evaluated in disseminated perinatal infections and encephalitis. Ara-A is an established herpes virus agent; It is active in preventing cytophthic effects in tissue cultures of HSV-1 and HSV-2.

In spite of the widespread occurrence and seriousness of herpes virus infections in the United States and around the world, no other treatments than IDU, trifluridine, Ara-A or acyclovir have been generally recognized as effective.

The present invention provides a treatment based on cholic acid and derivatives thereof. Cholic and deoxycholic acids, two bile acids structurally related to chenic acid, have been evaluated as antihyperlipidemic agents in human trials. Cholic acid has been shown to reduce serum cholesterol in hypercholesterolemic patients. Deoxycholic acid has been shown to decrease serum cholesterol levels in both normo-and hyperlipidemic patients. Chenodeoxycholic acid (chenic acid) is an effective agent for inducing dissolution of cholesterol gallstones, and has been reported in a number of clinical studies to significantly reduce plasma triglyceride levels in normal and hyperlipidemic patients. (See Burger's Medicinal Chemistry, 4th Edition, Part II, edited by Wolff, page 1248.)

Other therapeutic uses for these bile acids are disclosed in several U.S. Patents. U.S. Patent 4,241,047 to Lechevin et al discuss the use of chenic acid together with 4-methyl-7-hydroxycoumarine as an oral medicament for the treatment of biliary lithiasis. Frigerio U.S.P. 4,263,272 discloses a composition for oral use containing a bile acid suitable for lysis of calculii of cholesterol origin. Hilgermann et al U.S.P. 2,220,331 discuss the preparation of alkaline solutions of sodium taurocholate as a means for killing or destroying cocci, such as pneumococcus, gonococcus, meningococcus, streptococcus and similar cocci. Saltzman U.S.P. 4,029,775 also describes compounds related to cholic acid and derivatives, useful as antimicrobial compositions for oral use. The compounds are described as having antimicrobial, antibiotic and/or bacteriostatic properties. Wyatt et al U.S.P. 2,156,891 describe the use of bile acids for administration by injection to patients with arthritic and fibrositic disease.

None of these references suggest the use of bile acids or bile acid derivatives for the treatment of herpes simplex viral infections, nor do they suggest compositions adapted for topical administration for that use.

The present invention provides an effective and useful therapeutic treatment for herpes simplex viral infections of type 1 and type 2.

The invention thus relates to a composition for treating herpes virus infection in an animal which comprises a compound of the formula (I):

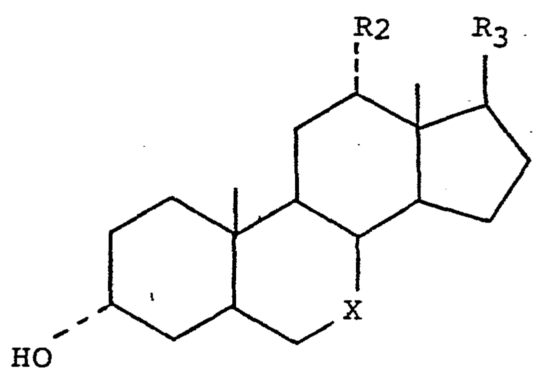

(I)

wherein X is >CH···$R_1$ or >C=0;

where $R_1$ and $R_2$ are the same or different and each is H or OH;

$R_3$ is $CH_3-\underset{|}{C}H\sim(CH_2)_n-COY$;

where n is 1, 2 or 3; and

Y is $OR_4$ or $NR_5R_6$,

where $R_4$ is H or a $C_1-C_{12}$ branched or unbranched alkyl group or a pharmaceutically acceptable cation;

$R_5$ and $R_6$ are the same or different and each is H, unsubstituted $C_1-C_{12}$ branched or unbranched alkyl, or branched or

unbranched $C_1$-$C_{12}$ alkyl substituted by $-CO_2R_4$ or by $-SO_3R_4$, or

$R_5$ and $R_6$ together with the nitrogen atom form a heterocyclic ring; together with a carrier suitable for topical administration.

The invention may be used in methods for the treatment of herpes simplex virus infections of the type 1 and type 2, especially by topical administration, and also relates to combinations of compounds of formula (I) with other anti-viral agents.

The compounds useful in this invention are those having the formula (I):

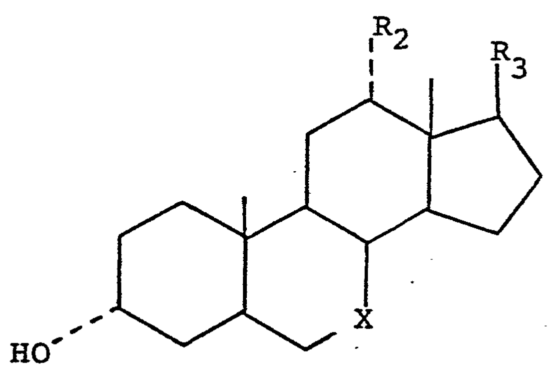

(I)

wherein X is $\rangle CH\cdots R_1$ or $\rangle C=0$;

where $R_1$ and $R_2$ are the same or different and each is H or OH;.

$R_3$ is $CH_3$ -CH~$(CH_2)_n$ -COY;

n is 1, 2 or 3;

Y is $OR_4$ or $NR_5R_6$, where

$R_4$ is H, a $C_1$ -$C_{12}$ branched or unbranched alkyl group or a pharmaceutically acceptable cation;

$R_5$ and R6 are the same or different and each is H , unsubstituted $C_1$ -$C_{12}$ branched or unbranched alkyl, or branched or unbranched $C_1$-$C_{12}$ alkyl substituted by -$CO_2R_4$ or by -$SO_3R_4$;

or $R_5$ and $R_6$ together with the nitrogen atom form a heterocyclic ring.

(In this and subsequent formulae, the stereochemical configuration around any asymmetric centre is indicated by conventional symbols: a solid line indicates "out of" the plane of the paper, a dotted line indicates "into" the plane of the paper, and a wiggle (~) indicates either configuration.)

Preferred are those compounds wherein n = 2.

Preferred are also those compounds where the alkyl groups are $C_1$-$C_6$; most preferred are lower alkyl groups, such as methyl, ethyl, propyl, butyl, isopropyl, and the like.

Among the pharmaceutically acceptable cations for the purpose of this invention are pharmaceutically acceptable metal cations, ammonium, amine cations or quaternary ammonium cations.

Especially preferred metal cations are those derived from the alkali metals e.g. lithium, sodium and potassium, and from the alkaline earth metals, e.g. magnesium and calcium, although cationic forms of other metals, e.g.aluminium, zinc and iron are within the scope of this invention.

Pharmacologically acceptable amine cations are those derived from primary, secondary or tertiary amines. Examples of suitable amines are methylamine, dimethyl amine, trimethyl amine, ethyl amine, dibutyl amine, triisopropyl amine, N-methylhexyl amine, decyl amine, dodecyl amine, allyl amine, crotyl amine, cyclopentyl amine, dicyclohexyl amine, benzyl amine, dibenzyl amine, alpha-phenylethyl amine, beta-phenylethyl amine, ethylene diamine, diethylene triamine, and like aliphatic, cycloaliphatic, and araliphatic amines containing up to and including about 18 carbon atoms, as well as heterocyclic amines, e.g. piperidine, morpholine, pyrrolidine, piperazine, and lower alkyl derivatives thereof, e.g. 1-methyl piperidine, 4-ethyl morpholine, 1-isopropyl pyrrolidine, 2-methyl pyrrolidine, 1, 4-dimethyl piperazine, 2-methylpiperidine, and the like; as well as amines containing water solubilizing or hydrophilic groups, e.g. mono- di-and triethanolamine, ethyldiethanolamine, N-butyl ethanolamine, 2-amino-1-butylol, 2-amino-2-ethyl-1, 3-propanediol, 2-amino-2-methyl-1-propanol, tris (hydroxymethyl) aminomethane, N-phenyl ethanolamine, N-(p-tert-amyl-phenyl) diethanolamine, galactamine, N-methylglucosamine, N-methylgalactosamine, ephedrine, phenylephrine, procaine, and the like.

Examples of suitable pharmacologically acceptable quaternary ammonium cations are tetramethyl ammonium,

tetraethyl ammonium, benzyltrimethyl ammonium, phenyltriethyl ammonium, and the like.

Compounds for which X is $>CH\cdots R_1$, where $R_1$ is hydrogen and R2 is OH, are derivatives of the deoxycholic acid family.

Compounds for which X is $>CH\cdots R_1$, where $R_1$ and $R_2$ are both OH, are derivatives of the cholic acid family.

Compounds for which X is $>C=0$ are derivatives of the 7-keto cholic acid family, e.g. 7-alpha-ketolithocholic acid.

Among the preferred compounds are those of the deoxycholic acid family.

Among the most preferred compounds of the present invention are those of the formula (II):

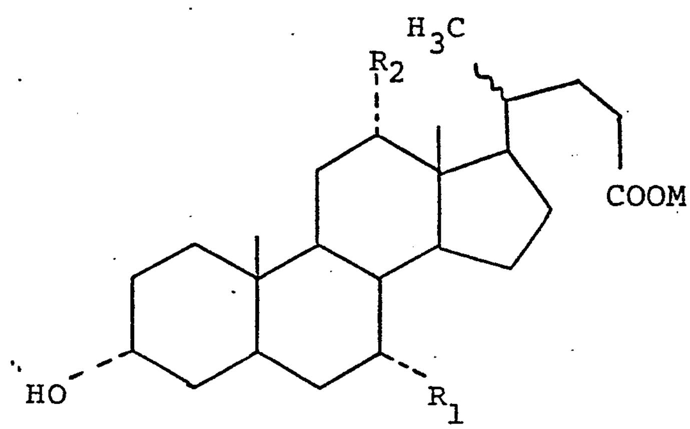

(II)

wherein $R_1$ and $R_2$ are as defined above and M is a pharmaceutically acceptable cation.

Also among the preferred compounds of the invention are those of the formula (III):

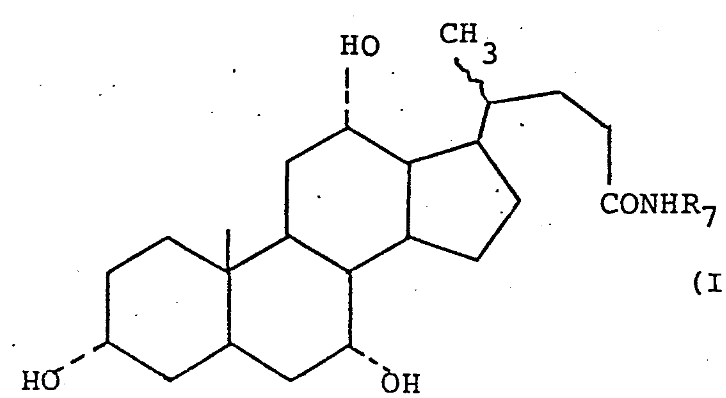

(III)

wherein $R_7$ is $-CH_2-CO_2R_4$ or $-CH_2CH_2SO_3R_4$. These are derivatives of glycocholic and taurocholic acids, respectively.

The heterocyclic amide derivatives of the invention comprise those heterocycles described supra in the listing of suitable cationic heterocyclic amines, i.e., piperidine, piperazine, pyrrolidine, aklyl derivatives thereof, and the like.

The compounds of this invention are either naturally occurring, since they are derivatives of the bile acids, or can be readily derivatized into the ketones, cationic salts, esters or amides by reactions well known to those of skill in the art. See, for example, Fieser and Fieser, Steroids (Reinhold, New York, 1959), or Dempsey, "Regulation of Steroid Biosynthesis" in Annual Reviews of Biochemistry, Vol. 43 (Annual Reviews Inc., Palo Alto, 1974). The bile acid compounds are also readily available from major chemical suppliers in the United States, such as Sigma Chemical Company (Saint Louis, Missouri) or Aldrich Chemical Company (Milwaukee, Wisconsin).

The compounds of this invention are useful in the treatment of viral infections derived from herpes simplex virus types 1 and 2. Conditions such as acute gingivostomatitis, rhinitis, keratoconjunctivitis, meningoencephalitis, eczema herpeticum, and traumatic herpes, such as herpetic whitlow and generalized cutaneous herpes simplex can be treated. Herpetic paronychia can also be treated. The compounds are also useful for the treatment of primary infection, chronic infection and clinical reactivations.

The compounds are preferably used in topical administration to areas of the body infected with HSV-1 and HSV-2. However, the compounds may also show activity when administered by the oral, intravenous, or intramuscular routes. Patients of any age or sex can be treated with the compounds.

Among the preferred topical carriers useful in preparing compositions according to this invention are those carriers generally known to the art, including inert materials such as medium chain-length fatty acids (e.g., $C_5$-$C_{20}$) or their esters, such as alcohols, celluloses, petroleum bases, and the like. For example, cream, gel, lotion or ointment bases can be used.

Among useful topical additives and carriers are cetyl esters wax, cetyl alcohol, white wax, gylceryl monostearate, propylene glycol monostearate, methylstearate, phenylethyl alcohol, sodium lauryl sulphate glycerine, mineral oil, white petrolatum, polyethylene, esters of mixed saturated fatty acids, stearyl alcohol, polysorbate 40, isopropylmyristate, cetyl stearyl alcohol, lanolin alcohol, disodium edetate, lactic acid, stearic acid, polyoxyl 40 stearate, polysorbate 60, sorbiton monostearate, liquid petrolatum, cocoa butter, polyethylene wax base with glyceryl monooleate or gylceryl monostearate, disodium monooleamidosulphosuccinate, citric acid, sorbic acid, preservatives such as methylparaben, propylparaben, or thimerosal, aromas such as mentholatum, as well as buffers and purified water. Water on its own would not generally be regarded as a topical carrier; neither would simple aqueous (for example alkaline) solutions.

Creams can be formulated to contain, for example, mono and di glycerides, squalene,polysorbate, cetyl

esters wax, steryl alcohol, sorbital solution or combinations thereof.

Ointments can preferably be prepared in while petrolatum.

Gels can be formulated for example with alcohol, carboxyvinyl polymer, propylene glycol, disodium edetate, diisopropanolamine or combinations thereof.

The compositions for either topical or other (for example intravenous) administrations will contain the active ingredient between 0.1 and no more than 90% by weight per volume, preferably 1 to 10% by weight per volume. When the administration is to be intravenous and for humans, the preparation will generally be sterile.

The dosage administered will be dependent upon the age, health and weight of the recipient, kind of concurrent treatment if any, frequency of treatment, and the nature of the effect desired. Generally, daily topical dosages of active ingredient compounds will be from about 0.01 to 10 ml volume of solution, gel, ointment or cream to the affected area, from one to several days, once or more daily or for the duration of the clinical infection.

The compounds of formula (I) may be used by themselves or in combination with an other antivirally effective compounds. In particular, a preferred composition according this invention comprises antivirally effective amounts of a combination of 1) a compound of formula (I), and 2) a compound of formula (IV):

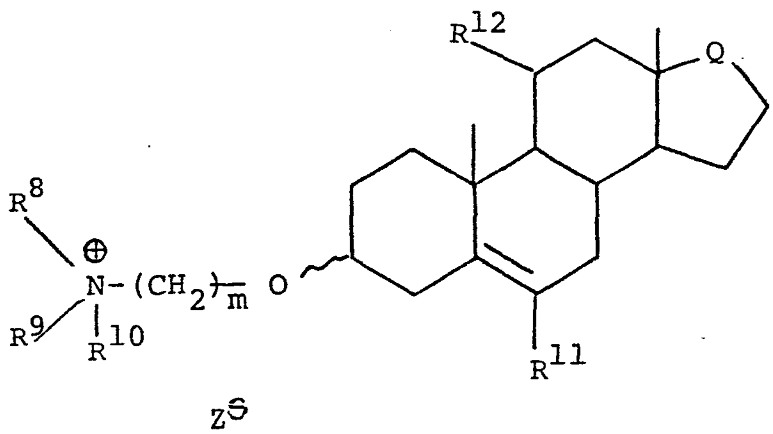

(IV)

wherein m is an integer from 2 to 6;

$R^8$ and $R^9$ are the same or different and each is H or a $C_1$-$C_{12}$ branched or unbranched alkyl group, or

$R^8$ and $R^9$ taken together with the nitrogen atom from a heterocyclic ring,

$R^{10}$ is H or a $C_1$-$C_{12}$ branched or unbranched alkyl group,

$R^{11}$ is H or a $C_1$-$C_3$ alkyl,

$R^{12}$ is H or HO,

Q is $\rangle C=O$, $\rangle N-OH$ or is

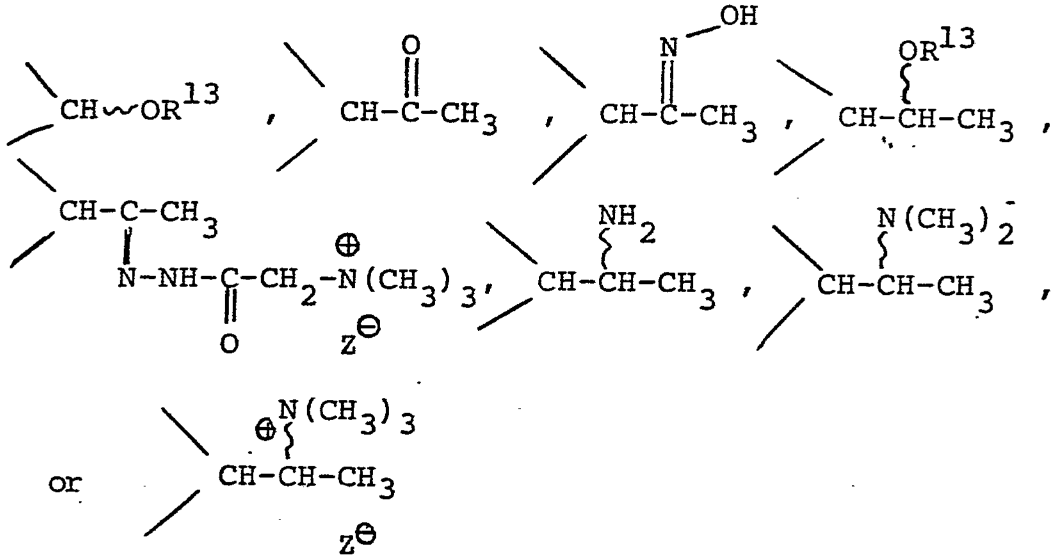

or

where $R^{13}$ is $R^8$ or $-\underset{\substack{\| \\ O}}{C}-R^8$; and

Z is a pharmaceutically acceptable anion.

Particularly preferred compositions are those in which, in the compound of formula I, X is $\rangle CH{-}{-}R_1$, $R_1$ is H and $R_2$ is OH particularly deoxycholic acid or a pharmaceutically acceptable cationic salt thereof; and

wherein the compound of formula IV is

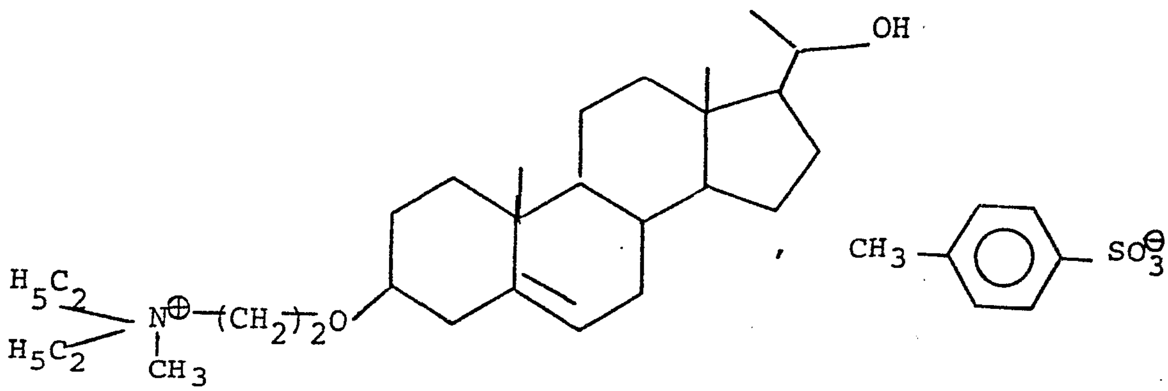

Alternatively, in such a composition, in the compound of formula I, X may be $\rangle CH{-}{-}R_1$, $R_1$ may be OH and $R_2$ may be OH.

Further preferred compositions are those in which, in the compound of formula I, X is $\rangle C{=}O$, $R_2$ is H; and wherein the compound of formula IV is

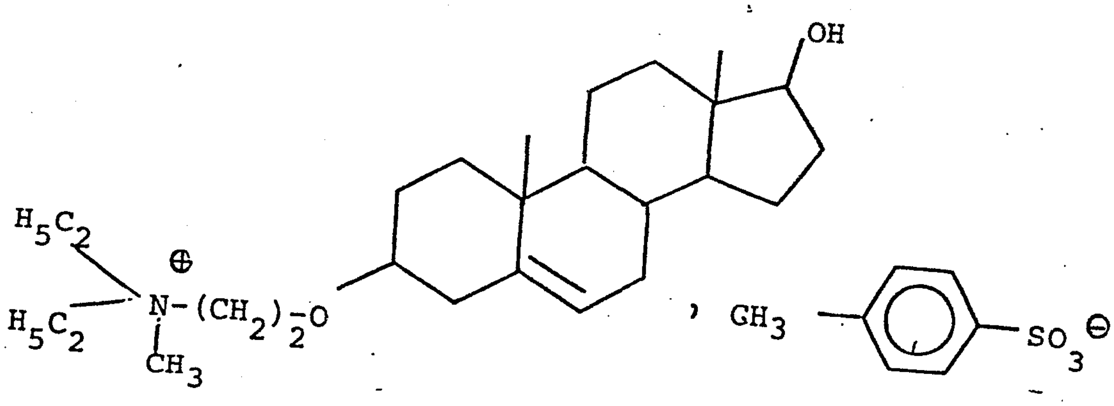

Pharmaceutically acceptable anions are, for example, chloride, bromide, iodide, sulphate, bisulphate, tartaric acid anion, succinate methylsulphonate, toluene sulphonate, benzenesulphonate, fluorosulphonate, p-nitrobenzenesulphonate, and the like.

The compounds of formula (IV) are described in French Patent No. 76/7320 and British Patent Specification No. 1,565,351 filed September 13, 1976, published April 16, 1980. Both of these publications are herein fully incorporated by reference.

Compounds of formula (IV) may be prepared by reacting the corresponding 3-hydroxy compound at various temperatures firstly with potassium tert-butylate or _n_-butyl lithium in an appropriate solvent (such as THF) and then with an aminoalkyl halide of the formula

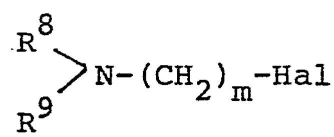

(in which $R^8$, $R^9$ and m have the meanings defined above and Hal is a halogen atom). The resultant 3-substituted derivative is then converted to the corresponding ammonium salt or quaternary ammonium derivative by reaction with an appropriate acid or quaternising agent.

The derivatives with 3α-stereochemistry can also be obtained by the substitution of the corresponding alcohols, or by alcoholysis of the $\Delta^5$ 3β-tosyloxy derivatives by the desired amino alcohols, with inversion of configuration at $C_3$, according to the method described by D.D. Evans and J. Hussey (J. Chem. Soc., 1968, 2504).

Compounds of forumula (IV) are disclosed therein as having anti-viral activity. This has been confirmed in the present invention with respect to at least the preferred compound TX047 (3-beta-[2-(Diethylamino)ethoxy]-20-beta-hydroxypregn-5-ene methyl p-toluenesulphonate) of the aforementioned two publications. TX047, both by itself and in combination with a compound of formula (I) is effective as an anti HSV-1 or HSV-2 agent.

A composition comprising both a compound of formula (I) and a compound of formula (IV) may contain between 0.1 and not more than 90% by weight per volume of the combination of both compounds in an inert carrier. The combination of both compounds may normally be formulated with any appropriate, inert pharmaceutical carrier, preferably a topical carrier, such as those described previously. The weight ratio of compound (I) to compound (IV) in the combination may vary (in pbw) from 1:10 to 10:1 of I:IV, most preferably about 1:1.

The modes of administration, dosages and other details of the treatment with the combination of compounds (I) and (IV) are like those described for the use of compound (I), supra.

Having now generally described this invention, the same will become better understood by reference to certain specific examples which are included herein for purposes of illustration only and are not intended to be limiting unless otherwise specified.

## Example 1

## Effect of Deoxycholic Acid in Vitro Against HSV-1 and HSV-2

Laboratory strains of HSV-1 and of HSV-2 having titres of a $10^8$ plaque forming units per millilitre were incubated with or without 5% (w/v) deoxycholic acid in buffered normal saline at 37 $^oC$ for 20 minutes. The samples were dialyzed against buffered normal saline at 4 $^o$ C for 12 hours, and then serial dilutions were assayed for lytic activity in the Vero cell line of African green monkey kidney cells. No lytic activity (ability to produce total cell lysis in confluent 30 mm dishes with Vero cells within 7 days of the infection) was observed with undiluted HSV-1 or HSV-2 treated with deoxycholic acid. Complete lysis was observed through $10^6$ (one million-fold) dilutions of the non-deoxycholate treated controls.

## Example 2

## Human Clinical Study Using DOC

A clinical study was performed in a classical double blind format with numbered vials containing either 1% sodium deoxycholic acid (DOC)in an aqueous

solution of 30% ethanol and 5% neopurcellin (a mixture of inert medium chain fatty acids). The placebo was exactly the same, except that it lacked the sodium deoxycholic acid.

Results are shown in the following Table 1:

TABLE 1

| Number (vials) | Number Patients Followed | Number Days To > 90% Improvement | Side Effects |
|---|---|---|---|
| 1 to 100 (TX047) | 93 | 1.774 | 2* |
| 101 to 200 (Placebo) | 42** | More patients did not follow treatment because lack of clinical improvement. 6.250 | |
| 201 to 300 (DOC) | 97 | 1.608 | 1* |

* irritation

**followed treatment - 58 patients did not show up to following visits.

<u>Patients treated with Tx047</u>: approx. 60% genital, 40% oral herpes. Efficacy was greatest in the treatment of oral infections and less but clearly active, and useful for genital herpes.

<u>Patients treated with placebo</u>: approx. 60% genital, 40% oral. A poor response was seen in the majority (> 80%) with prolonged and worsening symptoms and signs.

<u>Patients treated with DOC</u>: approx. 50% genital, 50% oral. Efficacy seen with genital herpes infections and also, although less dramatically, with oral herpes.

The findings indicate that both TX047 and sodium deoxycholic acid were able to markedly reduce the significant signs of herpes simplex infections including erythema, edema, vesiculation, ulceration and pain.

## Example 3

## Human Clinical Study Using a Combination of DOC and TX047

Compositions as in Example 2 were prepared except that placebo was compared to a combination formulation of 1% DOC and 1% TX047. The ratio of genital to oral herpes was about the same in the patients of this study than in the patients in the study of Example 2. Results are shown in the following Table 2:

TABLE 2

| Group | Number Patients Followed | Days > 90% Improvement | Side Effects |
|---|---|---|---|
| A) Placebo | 21 | 8 | None |
| B) (TX047 + DOC) | 24 | 1.575 | None |

The results indicate that the combination TX047 plus DOC is superior to the use of TX047 alone (Example 2) of DOC alone (Example 2).

## Example 4

Compositions containing 0.5-2% w/v of TX047 and 1-3% w/v of deoxycholic acid, sodium salt, were formulated in an aqueous solution of 30% ethanol and 5% neopurcellin. Such compositions when applied topically suppressed HSV-1 and HSV-2 infections in human patients.

Having now fully described this invention it will be apparent to those of ordinary skill in the art that the same can be performed within a wide and equivalent range of compositions, dosages, administrations, compounds and HSV-1- and HSV-2-related infections, without affecting the spirit or scope of the invention or of any embodiment thereof.

CLAIMS:

1. A composition adapted for topical treatment of herpes simplex virus infection which comprises an antivirally effective amount of a compound of the formula:

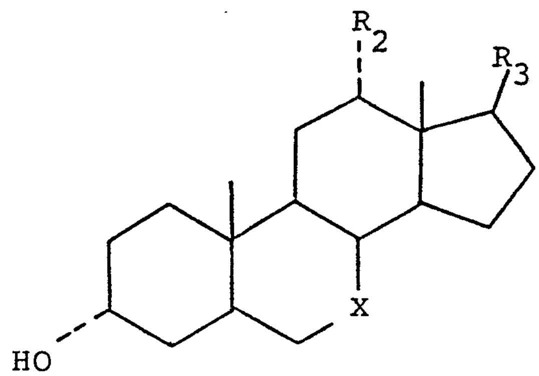

wherein X is $>CH-R_1$ or $>C=0$;

where $R_1$ and $R_2$ are the same or different and each is -H or -OH;

$R_3$ is $CH_3-CH-(CH_2)_n-COY$;

n is 1, 2 or 3;

Y is $OR_4$ or $NR_5R_6$, where

$R_4$ is H, a $C_1$-$C_{12}$ branched or unbranched alkyl group or a pharmaceutically acceptable cation;

$R_5$ and $R_6$ are the same or different and each is H, unsubstituted $C_1$-$C_{12}$ branched or unbranched alkyl, and branched or unbranched $C_1$-$C_{12}$ alkyl substituted by $-CO_2R_4$ or by $-SO_3R_4$;

or $R_5$ and $R_6$ together with the nitrogen atom form a heterocyclic ring;

together with a pharmaceutically inert topical carrier.

2. A composition as claimed in Claim 1 wherein the inert topical carrier comprises a fatty acid or ester of medium chain length.

3. A composition as claimed in Claim 1 or 2 which is in the form of a gel, an ointment, a cream or a lotion.

4. A composition as claimed in Claim 1 wherein the compound is deoxycholic acid or its pharmaceutically acceptable cationic salts.

5. A composition useful for treatment of herpes simplex virus infection which comprises

a) A compound of the formula (I):

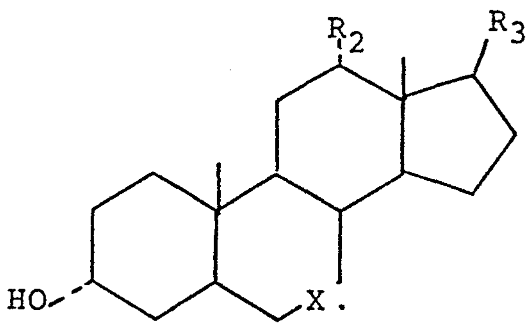

(I)

wherein X is $>CH{-}{-}R_1$ or $>C=0$;

where $R_1$ and $R_2$ are the same or different and are -H or -OH;

$R_3$ is $CH_3-CH\sim(CH_2)_n-COY$;

n is 1, 2 or 3;

Y is $OR_4$ or $NR_5R_6$, where

$R_4$ is H, a $C_1-C_{12}$ branched or unbranched alkyl group or a pharmaceutically acceptable cation;

$R_5$ and $R_6$ are the same or different and each is H, unsubstituted $C_1-C_{12}$ branched or unbranched alkyl, or branched or unbranched $C_1-C_{12}$ alkyl substituted by $-CO_2R_4$ or by $-SO_3R_4$;

or $R_5$ and $R_6$ together with the nitrogen atom for a heterocyclic ring; and

b) a compound of formula (IV):

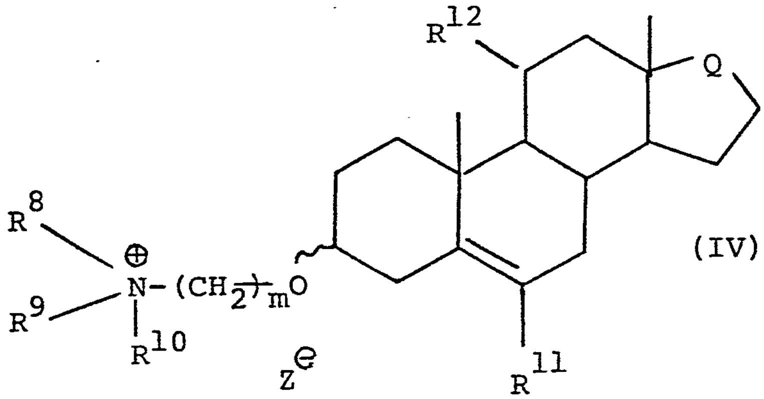

(IV)

wherein m is an integer from 2 to 6;

$R_8$ and $R_9$ are the same or different and each is H or a branched or unbranched $C_1$-$C_{12}$ alkyl group, or

$R_8$ and $R_9$ taken together with the nitrogen atom a heterocyclic ring;

$R_{10}$ is H or a branched or unbranched $C_1$-$C_{12}$ alkyl group;

$R_{11}$ is H or a $C_1$-$C_3$ alkyl group;

$R_{12}$ is H or HO;

Q is 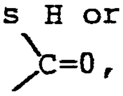, 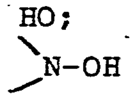 or is

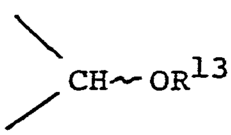 , 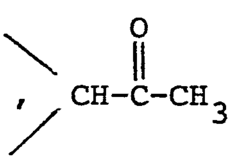 , 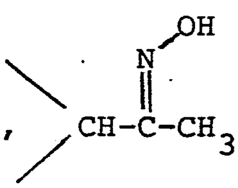 , 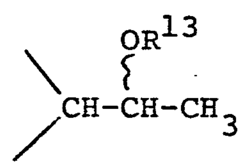 ,

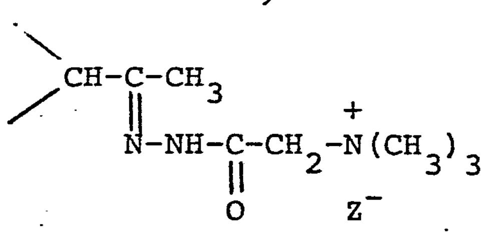 , 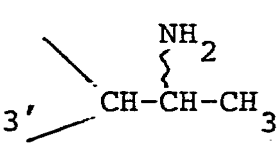 , 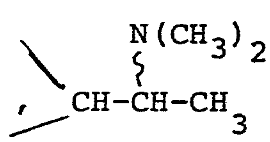 ,

or 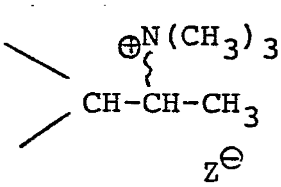

where $R^{13}$ is $R^8$ or 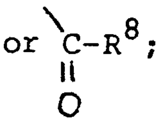; and

Z is a pharmaceutically acceptable anion.

6. A composition as claimed in Claim 5 which also contains an inert pharmaceutical carrier.

7. A composition as calimed in Claim 5 or 6 which is adapted for topical administration.

8. A compound of the formula:

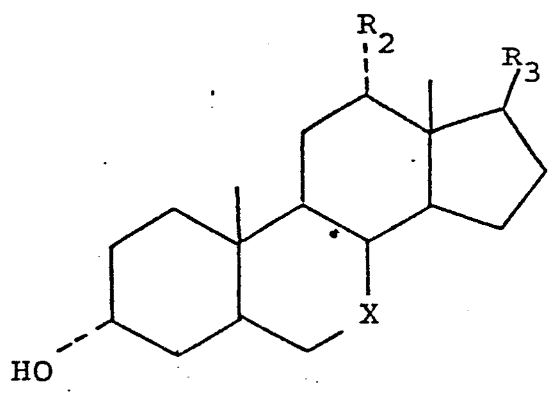

wherein X is $>CH{-}{-}R_1$ or $>C=O$;

where $R_1$ and $R_2$ are the same or different and each is -H or -OH;

$R_3$ is $CH_3-CH-(CH_2)_n-COY$;

n is 1, 2 or 3;

Y is $OR_4$ or $NR_5R_6$, where

$R_4$ is H, a $C_1$-$C_{12}$ branched or unbranched alkyl group or a pharmaceutically acceptable cation;

$R_5$ and $R_6$ are the same or different and selected from the group consisting of H, unsubstituted $C_1$-$C_{12}$ branched or unbranched alkyl, and branched or unbranched $C_1$-$C_{12}$ alkyl substituted by $-CO_2R_4$ or by $-SO_3R_4$;

or $R_5$ and $R_6$ together with the nitrogen atom form a heterocyclic ring;

for use in antiviral, especially antiherpes, chemotherapy.

9. A product containing

a) a compound of the formula (I):

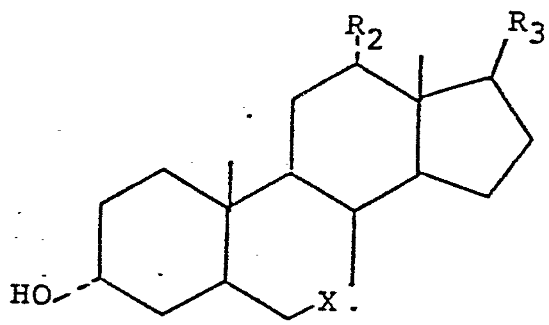

(I)

wherein X is $>CH-R_1$ or $>C=O$;

where $R_1$ and $R_2$ are the same or different and each is -H or -OH;

$R_3$ is $CH_3-\overset{OH}{\overset{|}{C}H}\sim(CH_2)_n-COY$;

n is 1, 2 or 3;

Y is $OR_4$ or $NR_5R_6$, where

$R_4$ is H, a $C_1$-$C_{12}$ branched or unbranched alkyl group or a pharmaceutically acceptable cation;

$R_5$ and $R_6$ are the same or different and

each is H, unsubstituted $C_1-C_{12}$ branched or unbranched alkyl, or branched or unbranched $C_1-C_{12}$ alkyl substituted by $-CO_2R_4$ or by $-SO_3R_4$;

or $R_5$ and $R_6$ together with the nitrogen atom for a heterocyclic ring; and

b) a compound of formula (IV):

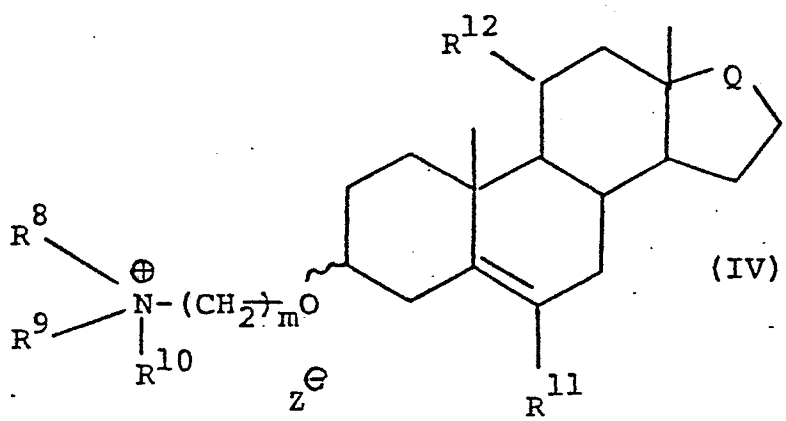

(IV)

wherein m is an integer from 2 to 6;

$R_8$ and $R_9$ are the same or different and each is a branched or unbranched $C_1-C_{12}$ alkyl group, or

$R_8$ and $R_9$ taken together with the nitrogen atom for a heterocyclic ring;

$R_{10}$ is H or a branched or unbranched $C_1-C_{12}$ alkyl group;

$R_{11}$ is H or a $C_1-C_3$ alkyl group;

$R_{12}$ is H or HO;

Q is $>$C=0, $>$N-OH or is

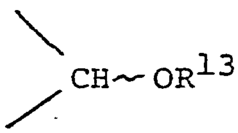 , 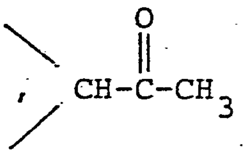 , 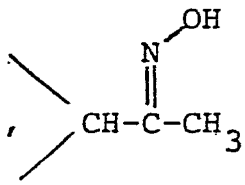 , 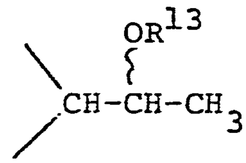 ,

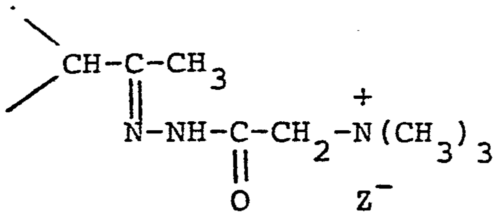 , 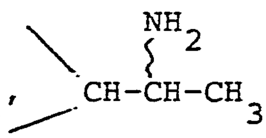 , 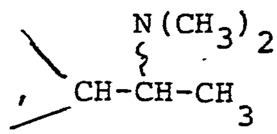 ,

or 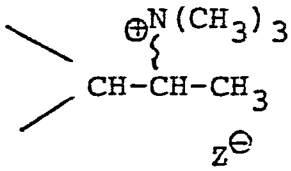

where $R^{13}$ is $R^8$ or 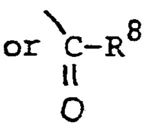; and

Z is a pharmaceutically acceptable anion,

as a combined preparation for simultaneous, separate or sequential use in antiviral chemotherapy.

10. A sterile injection preparation of a composition as claimed in Claim 5 or a compound as defined in Formula (I).